Europäisches Patentamt

European Patent Office

Office européen des brevets

(19)

(11) Publication number: **0 110 706**
A2

(12) **EUROPEAN PATENT APPLICATION**

(21) Application number: **83307254.9**

(22) Date of filing: **29.11.83**

(51) Int. Cl.³: **A 61 K 39/395**, A 61 K 39/42

(30) Priority: **29.11.82 GB 8233919**

(43) Date of publication of application: **13.06.84**
**Bulletin 84/24**

(84) Designated Contracting States: **CH DE FR GB IT LI NL**

(71) Applicant: **Baylor College of Medicine, Texas Medical Center 1200 Moursund Avenue, Houston Texas 77030 (US)**

(72) Inventor: **Kennedy, Ronald Curtis, 5500 N. Braeswood, No. 279, Houston Texas 77096 (US)**
Inventor: **Dreesman, Gordon Ronald, 6704 Community Drive, Houston Texas 77005 (US)**
Inventor: **Melnick, Joseph Louis, 8838 Chatsworth, Houston Texas 77024 (US)**

(74) Representative: **Lucas, Brian Ronald et al, Lucas, George & Co. 135 Westhall Road, Warlingham Surrey CR3 9HJ (GB)**

(54) **Injection of anti-idiotype to elecit immune response to infectious viral agents.**

(57) Anti-ID antibodies modulate the immune response to HBsAg. Injection of anti-ID antibodies alone induce anti-HBs that are directed against the a determinant and inhibit a common interspecies anti-HBs ID-anti-ID reaction. In addition, anti-ID treatment prior to HBsAg on synthetic peptide stimulation enhance the anti-HBs response. Thus, the immune response to HBV may be controlled via idiotype networks with anti-ID antibodies as vaccine candidates by modulating the immune response to an infections viral agent.

A. Reaction of idiotype with specific epitope (HBs peptide)

Anti-HBs + HBs Peptide → Immune Complex

B. Blocking of idiotype with anti-idiotype antibody

Anti-HBs + Anti-CId → + Free Peptide

EP 0 110 706 A2

0110706

-1-

## INJECTION OF ANTI-IDIOTYPE TO ELICIT IMMUNE RESPONSE TO INFECTIOUS VIRAL AGENTS

The invention described herein was made during the course of work under grants from the National Heart, Lung and Blood Institute, National Institutes of Health, and the United States Army Medical Research and Development Command.

## Cross-References to Related Applications None.

## Prior Art Statement and Background of the Invention

The following is an explanation of the background of the present invention together with a listing which, in the opinion of the Applicants, sets forth the closest prior art of which the Applicants are aware. A concise explantion of the relevance of the more important items is included.

Viral hepatitis has assumed significant world-wide epidemic proportions. It is estimated that there are two hundred million carriers of hepatitis B virus ("HBV") world-wide. The development of a conventional vaccine has been hampered by the inability to grow hepatitis B virus in tissue culture. As a result, it has been necessary to produce hepatitis B subunit particle vaccines by isolating and purifying the 22 nm lipoprotein particles composed of hepatitis B surface antigen ("HBsAg") from plasmas of asympotomatic human carriers. However, such formalin- or heat-inactivated vaccines have the disadvantages of substantial expense and limited supply. In addition, such a source presents potential hazards in view of unknown factors that may be present in the plasma. Also, as high-risk populations are immunized, sources of plasma containing large quantities of HBsAg will become scarce.

The concept that the immune response to an antigen can be regulated via an idiotype network was first proposed by Niels Jerne in 1974. Idiotypes (distinct antigenic determinants), located on or close to the antigen-binding site of both antibody molecules and lymphocyte antigen receptors, are the components of this network. Within this framework a series of idiotype-anti-idiotype reactions may potentiate or suppress the network and control the immune response of an individual to antigen. Some theoretical implications of anti-idiotype antibodies as potential vaccines for infectious agents have been discussed by A. Nisonoff and E. Lamoyi. Anti-idiotypes that recognize the antigen-binding site of idiotype molecules share structural homologies with the antigen that induced the idiotype. Therefore, anti-idiotypes (secondary antibodies) may represent an internal image of the antigen so that injection of anti-idiotypes may induce primary antibodies (idiotype) without classical antigenic stimulation.

As pointed out above, the possibility of anti-idiotype antibodies as potential vaccines for infectious agents have been suggested previously. The following is a list of references relating to such vaccines or processes with respect thereto and which will be referred to herein by respective reference numbers.

1. Kunkel,H.G., M. Mannik, and R. C. Williams. 1963. Science 140:1218.

2. Oudin, J., and M. Michel. 1963. C.R.Acad.Sci.(Paris) 257:805.

3. Jerne, N.K. 1974. *Ann. Immunol.* (Paris) 125c: 373-389.

4. A. Nisonoff, E. Lamoyi, *Clin. Immunol. Immunopath.* 21, 397 (1981).

5. A. D. Strosberg, *Springer Semin. Immunopath.* 6, 67 (1983).

6. D. L. Sacks, G. H. Kelsoe, D. H. Sachs, *ibid.* 6, 79 (1983).

7. Cosenza, H. and Kohler, H. *Proc. natn. Acad. Sci. U.S.A.* 69, 2701-2705 (1972).

8. Eichmann, K. *Eur. J. Immunol.* 4, 296-302 (1974).

9. Hart, D.A., Wang, A. L., Pawlak, L. L. & Nisonoff, A. *J. exp. Med.* 135, 1293-1300 (1972).

10. Bluestone, J. A., Sharrow, S. O., Epstein, S. L., Ozato, K. & Sachs, D. H. *Nature* 291, 233-235 (1981).

11. Cazenave, P. A. *Proc. natn. Acad. Sci. U.S.A.* 74, 5122-5125 (1977).

12. Kelsoe, G., Reth, M. & Rajewsky, K. *Immunol. Rev.* 52, 75-88 (1980).

13. Sachs, D. H., El-Gamil, M. & Miller, G. *Eur. J. Immunol.* 11, 509-516 (1981).

14. Trenkner, E. & Riblet, R. *J. exp. Med.* 142, 1121-1135 (1975).

15. Urbain, J., Wikler, M., Franssen, J. D. & Collignon, C. *Proc. natn. Acad. Sci. U.S.A.*, 74, 5126-5130 (1977).

16. Kennedy, R.C. and Dreesman, G.R. *J. Immunol.* 130:385-389, 1983.

17. Kennedy. R.C., Sanchez, Y., Ionescu-Matiu, I., Melnick, J.L. and Dressman, G.R. Virology 122:219-221, 1982.

18. Kennedy, R.C., Ionescu-Matiu, I., Sanchez, Y. and Dreesman, G.R. *Eur. J. Immunol.*, 1983.

19. Kennedy, R.C., Dreesman, G.R., Sparrow, J.T., Culwell, A.R., Sanchez, Y., Ionescu-Matiu, I., Hollinger, F.B. and Melnick, J.L. *J. Virol.* 46:653-655, 1983.

20. Ionescu-Matiu, I., Kennedy, R.C., Sparrow, J.T., Culwell, A.R., Sanchez, Y., Melnick, J.L. and Dreesman, G.R. J. Immunol. 130:1947-1952, 1983.

21. Kennedy, R.C. and Dreesman, G.R. J. Virol. Meth. 7:103-115, 1983.

22. D.L. Sacks, K.M. Esser, A. Sher J. Exp. Med. 155, 1108 (1982).

23. D.L. Sacks, A. Sher J. Immunol. 131, 1511 (1983).

24. Emini, E.A., Jameson, B.A. & Wimmer, E. Nature 304, 699-703 (1983).

The observation of a unique antigenic determinant located on or close to the antigen combining site of a specific antibody molecule was originally reported in references 1 and 2. This determinant was termed an immunoglobulin idiotype (ID). ID, along with homologous anti-ID antibodies was theorized to be components of a network of complex reactions that regulate a given immune response as originally reported in reference 3. References 4, 5 and 6 suggest several of the theoretical implications involved with the possible use of anti-ID antibodies as potential vaccines for infectious diseases. A number of studies with experimental model systems have illustrated the applicability of the above concepts that anti-ID antibodies can manipulate the immune response. Injection of anti-ID antibodies followed at a later date with injection of antigen has resulted in either suppression of ID-positive antigen-binding molecules (references 7-9) or an increased ID expression and antigen-binding activity (references 10-15).

The impetus to study idiotypes associated with antibody to hepatitis B surface antigen (anti-HBs) came from the knowledge that anti-HBs was protective against HBV infection. We initially generated four rabbit anti-idiotype antisera against affinity purified anti-HBs from two different individuals. Each of the four anti-idiotype reagents detected a common anti-HBs idiotype (reference No. 16). A single anti-idiotype antiserum was selected for further study. The common human idiotype was detected in purified anti-HBs from three individuals and also in anti-HBs-positive sera obtained from six hemophilic patients. The ability of both HBsAg and a virus-derived HBsAg native polypeptide to inhibit the idiotype-anti-idiotype reaction suggested the anti-HBs idiotype was associated with the antibody-combining site. Idiotype determinants were detected because the anti-idiotype antisera did not interact effectively with IgG preparations from: (i) the idiotype donor after removal of anti-HBs; (ii) a pool of human sera negative for anti-HBs; and (iii) an individual with a high level of antibody to herpes simplex virus. Attesting further to the idiotypic specificity was the inability of the anti-idiotype to bind either HBsAg or the native HBsAg-derived polypeptide. These data indicated that we were detecting an antibody-combining site associated, common human anti-HBs idiotype (Reference No. 16).

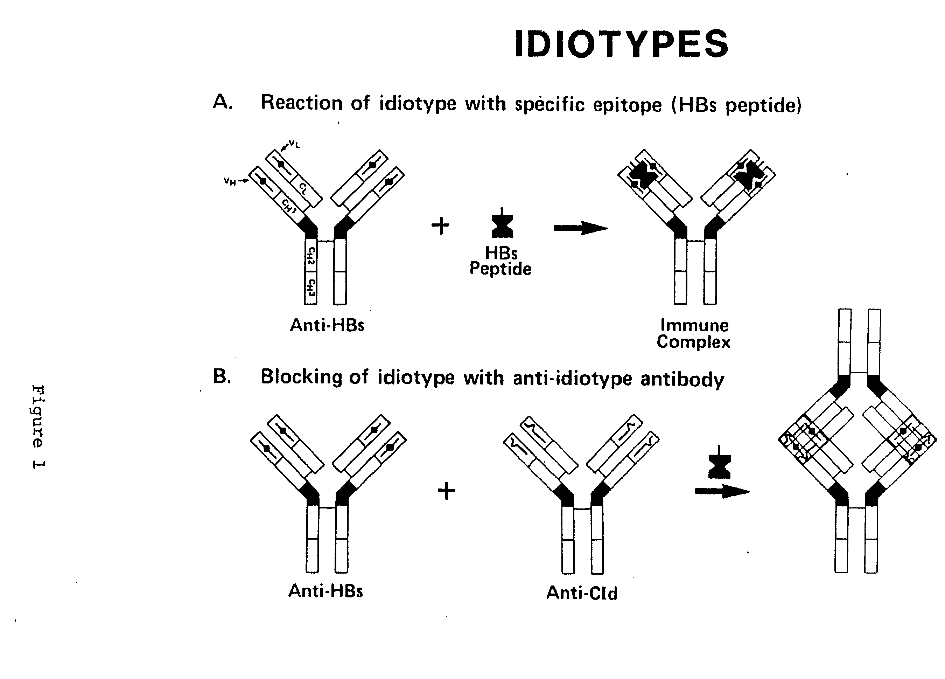

Further characterization of the common idiotype revealed that it was induced by the group a determinant because three HBsAg preparations purified from three pools of human plasma positive for HBsAg adw, ayw or adr subtypes inhibited the idiotype-anti-idiotype reaction equally on a weight basis. Figure 1. We also tested the ability of HBsAg-derived polypeptides to inhibit the idiotype-anti-idiotype reaction and found that sodium dodecyl sulfate-denatured HBsAg viral polypeptides virtually lost their capacity to inhibit when compared to the native polypeptides. Also reduction of the disulfide bonds and alkylation of free thiol groups destroyed the ability of the native HBsAg-derived polypeptide to inhibit the idiotype-anti-idiotype reaction. These data suggested that the common anti-HBs idiotype was directed against a conformation-dependent group-specific a epitope (Reference No. 17).

We have recently characterized the expression of the common idiotype on anti-HBs produced in rabbits, guinea pigs, swine, goats, chimpanzees, and BALB/c mice that had been immunized with HBsAg. Expression of the idiotype in sera from the various species was associated with anti-HBs molecules. We also determined that anti-HBs from chickens successfully immunized with HBsAg failed to express the common idiotype (Reference No. 18).

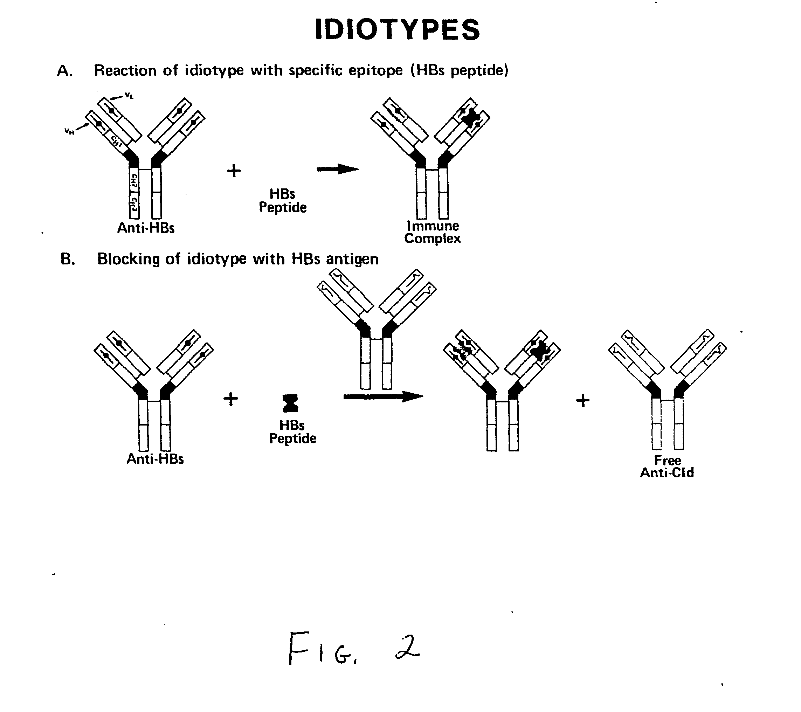

We tested the ability of a cyclic synthetic peptides that contained amino acid residues 122-137 (Peptide 1) and 117-137 (Peptide 2) analogous to P25 to inhibit the common idiotype-anti-idiotype reaction. Figure 2. On a molar basis these peptides were $10^3$-fold less efficient than intact HBsAg in inhibiting the idiotype-anti-idiotype reaction. The inability of the peptides to compete equally on a molar basis with HBsAg indicates that this peptide does not represent the complete a determinant and suggests other amino acid sequences are also important in defining the complete a epitope. The inhibition of the idiotype-anti-idiotype reaction by cyclic peptide 122-137 suggests that this sequence is related to antigenic determinants responsible for eliciting a population of human anti-HBs expressing a common idiotype. The importance of conformation was again demonstrated by reducing the disulfide bond in peptide 1 and alkylating the free thiol groups. This destroyed the ability of peptide 1 to inhibit the idiotype-anti-idiotype reaction (Reference No. 19). We have shown that mouse monoclonal antibodies which react with cyclic peptide 122-137 also inhibit the ID-anti-ID reaction whereas anti-HBs monoclonal which do react with the cyclic peptide fail to inhibit the ID-anti-ID reaction (Reference No. 20).

Detailed description of the prepartion of anti-ID antibodies and their characterization are given in reference No. 21.

The only other experimental evidence to our knowledge that anti-ID can induce protective immunity against an infectious agent has been reported for African trypanosomiasis in mice (References 22 and 23). These workers used mouse monoclonal anti-ID reagents.

Reference 24 is a description of priming an immune response to polio virus by prior injection of a polio synthetic peptide.

## Summary of the Invention

The present invention is directed to (1) a new composition of matter; (2) a composition for use in an immunizing preparation; (3) a composition for eliciting production of antibody to hepatitis B surface antigen (HBsAg); (4) a method for eliciting production of antibody HBsAg; (5) a method for priming the immune response prior to a subsequent inoculation of HBsAg; and (6) a method for priming the immune response prior to a subsequent inoculation of HBsAg synthetic peptide.

## Brief Description of the Drawings

Figure 1 graphically depicts inhibition of a human anti-HBs ID-anti-ID reaction with HBsAg subtypes adw, ayw and adr,

Figure 2 graphically depicts inhibition of anti-HBs, ID with HBsAg with anti-ID antibody,

Figure 3 graphically depicts that a mouse responds by production of anti-HBs in response to an injection of HBsAg or with an anti-ID reagent,

Figure 4 graphically depicts kinetics of the anti-HBs plaque-forming units (PFU) response in mice injected with antibody to idiotype followed later with an injection of HBsAg.

## Detailed Description of the Preferred Embodiment

Rabbit immunoglobulin G (IgG) anti-serum to the idiotype was exhaustively adsorbed to remove isotypic and allotypic antibodies prior to affinity purification by acid elution from a CNBr-activated Sepharose 4B column coupled to the common human anti-HBs idiotype. The anti-idiotype nature of this rabbit antiserum has been described previously. An IgG fraction obtained from the serum of the rabbit before injection of the idiotype served as a control antibody preparation. We determined the antibody concentration of the two preparations in a spectrophotometer at 280 nm using an extinction coefficient of 15 for a 1 percent preparation. For the immunogen, we used nondenatured HBsAg-derived polypeptide, isolated as highly antigenic micelles.

To quantitate the anti-HBs immune response at the cellular level, Jerne hemolytic plaque assays were performed in which IgM-secreting cells were measured by direct plaques and IgG-secreting cells by indirect plaques. Briefly tannic acid-treated sheep red blood cells (SRBC) were coated with HBsAg (subtype _ayw_) and used as the indicator cells in the plaque assay. Passive hemagglutination was used with mouse anti-HBs to ensure the presence of HBsAg on the SRBC. Spleen cell suspensions from immunized BALB/c mice were mixed with HBsAg-coated SRBC in agarose. After incubation for one hour at 375° C., SRBC-adsorbed guinea pig complement was added. After two hours at 37°C., the direct hemolytic plaques due to secretion of IgM antibodies were counted and the number of PFU per spleen was calculated from the total number of cells per spleen. Indirect plaques due to IgG-secreting cells were determined by adding a serum containing rabbit antibodies to mouse IgG prior to the addition of complement. Both uncoated SRBC and tannic acid-treated SRBC coated with ovalbumin served as controls in each experiment.

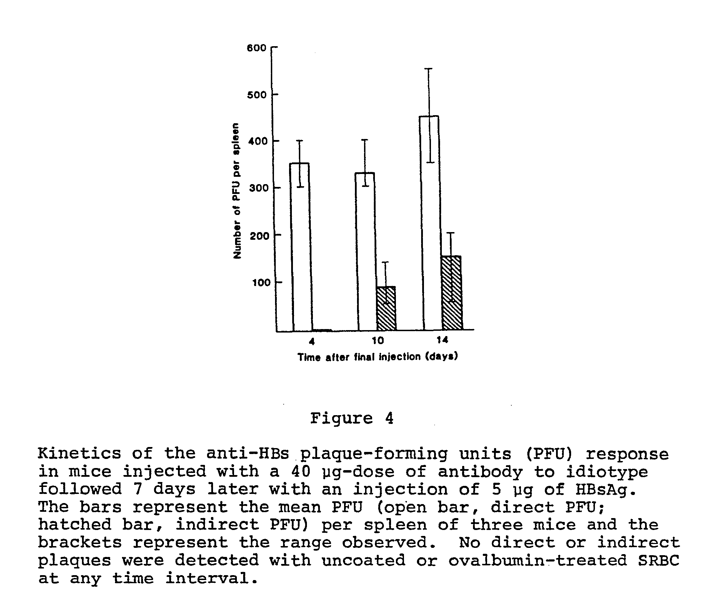

To establish the time interval after HBsAg injection when an optimal anti-HBs response was noted, it was necessary to determine the kinetics of the anti-HBs PFU response. In the first experiment reported graphically in Figure 4, BALB/c mice were immunized with 40 µg of purified antibodies to the idiotype in saline and then, 7 days later,

with 5 µg of HBsAg. Three animals were killed at 4, 10, and 14 days after the HBsAg injection, the spleens were removed, and the number of PFU per spleen was determined. From the data in Figure 4, it was apparent that the direct anti-HBs PFU response did not differ significantly at the three time intervals examined; however, no indirect plaques were detected on day 4. That the anti-HBs-secreting cells were specific was demonstrated by the fact that no direct or indirect plaques were detected with either uncoated or ovalbumin-coated SRBC at any of the three time intervals. Fourteen days after the mice received their final inoculum was the time interval selected to perform the remaining experiments.

Five groups of BALB/c mice were treated with antibodies to the idiotype, IgG from nonimmunized rabbits, or HBsAg, and their spleen cells were assayed for PFU response 14 days after the final injection. Injection of antibodies to the idiotype prior to HBsAg exposure generated a significantly higher number of direct PFU (531 as opposed to 183) when compared to mice inoculated with IgG from nonimmunized rabbits (based on a two-tail t-test, $P < 0.05$; Table 1).

-10-　　0110706

TABLE 1. Anti-HBs response expressed as PFU (mean ± standard error of the mean) per spleen. Each group of six mice received 40 µg of antibody to the idiotype or IgG from a nonimmunized rabbit (pre-IgG) or 5 µg of HBsAg on day 0, followed by the indicated injections on day 7, all by the intraperitoneal route. Spleens were removed on day 21. Cells from each mouse spleen were assayed in duplicate for the presence of both direct and indirect anti-HBs PFU. No hemolytic plaques were obtained with any spleen cells when uncoated or ovalbumin-coated SRBC were used as controls.

| First injection | Second injection | Number of Mice | Direct (IgM-secreting cells) | Direct (IgG-secreting cells) |
|---|---|---|---|---|
| Antibody to Idiotype | HBsAg | 7* | 531 ± 94** | 178 ± 29 |
| Pre-IgG | HBsAg | 8 | 183 ± 65 | 125 ± 36 |
| Antibody to idiotype | Antibody to idiotype | 6 | 66.7 ± 47.8 | 100 ± 29 |
| Pre-IgG | Pre-IgG | 6 | 16.7 ± 16.7 | 8.3 ± 8.3 |
| HBsAg | HBsAg | 6 | 167 ± 36 | 1683 ± 168 |

*One mouse died during the experiment. **Statistical methods, including the two-tail Student's t-test and a single-factor analysis of variance by ranks test, the Kruskal-Wallis test, also indicated direct PFU obtained in the group receiving antibody to idiotype before HBsAg was significantly different from all other groups of mice at P = 0.05.

The increase in the mean direct PFU detected in the second group of mice receiving antibodies to the idiotype before HBsAg injection (Table 1) appears to reflect an increase in sample size when compared to the time interval experiment (Figure 4) (531 from Table 1 as opposed to 450 from Table 4). The numbers of indirect PFU obtained in mice that received antibodies to the idiotype or preimmune IgG prior to HBsAg were ten times lower when compared to mice given two injections of HBsAg. However, the group receiving both antibodies to the idiotype and HBsAg did produce a higher number of direct PFU when compared to the group given HBsAg only (531 compared to 167; Kruskal-Wallis, $P < 0.05$). The number of indirect IgG anti-HBs PFU per spleen in the group receiving two HBsAg injections was on the same order of magnitude as those numbers reported for PFU per spleen in secondary responses to HBsAg and other viral antigens, thus giving validity to our hemolytic plaque assay as a method for quantitating anti-HBs-secreting cells.

Injection of antibodies to idiotype alone increased by 12 times the number of indirect PFU with anti-HBs specificity when compared to mice that received only preimmune IgG (100 compared to 8.3; Kruskal-Wallis, $P < 0.05$; Table 1). Although the number of direct PFU was higher in the mice that were injected with antibodies to idiotype alone, the number was not significantly different from that obtained with the group treated with nonimmunized rabbit IgG ($P > 0.1$). The specificity of the PFU response was again demonstrated as no plaques were detected with either uncoated or ovalbumin-coated SRBC in either the direct or indirect assay. The reason for the detection of anti-HBs PFU in only one of six mice that received nonimmunized rabbit IgG for both injections is now known; however, this resulted in the standard error and the mean being equal. The numbers of direct and indirect PFU from the one mouse in this group were low and are probably not meaningful.

Although the standard deviation also exceeded the mean for direct PFU in groups treated with either antibodies to idiotype alone or with nonimmunized rabbit IgG and HBsAg, single-factor analysis of variance by ranks [Kruskal-Wallis test] indicated that mean ranks of direct PFU differed significantly ($P < 0.005$) from the group receiving antibodies to idiotype and HBsAg. Such analysis was necessary because of the heterogeneity of the variances within the individual groups. The significance levels obtained by both the parametric two-tail Student's t-test and the nonparametric Kruskal-Wallis test were consistent.

Thus the injection of antibodies to idiotype enhanced the anti-HBs response at the cellular level. An increased number of IgM anti-HBs-secreting cells was obtained by injecting antibodies to the idiotype before antigen exposure. The reason for the increase of cells secreting IgM, but not those secreting IgG, is not known. However, it may reflect the recruitment, by the antibodies to the idiotype, of accessory cells that aid in the induction of a primary anti-HBs response, thus increasing the number of IgM anti-HBs-secreting cells.

Consequently, the presence of anti-ID induced anti-HBs activity in serum of BALB/c mice was investigated to ascertain whether these antibodies expressed an interspecies ID that would be indicative of an ID-anti-ID controlled network.

The first experiment was performed to determine the most immunogenic form(s) of anti-ID antibodies for enhancing the anti-HBs response. Our previous study used soluble anti-ID antibodies in the modulation of the murine anti-HBs response at the spleen cell level; however, other investigators demonstrated that high levels of antibody could be induced in their ID systems without antigen stimulation by injecting cross-linked anti-ID. Because higher titers of anti-HBs were generated in experimental animals when HBsAg was alum-precipitated rather than in aqueous solution, compared the effects on the anti-HBs response by injecting alum-precipitated material versus soluble anti-ID antibodies prior to HBsAg stimulation. As shown in Table 2, the mean anti-HBs titer was higher in groups of mice receiving alum-precipitated anti-ID when compared to anti-ID in saline prior to HBsAg injection (solid phase radioimmunoassay, AUSAB, Abbott Laboratories, N. Chicago, Il.; AUSAB titers of 487.5 versus 72.5). Only 1 of 4 mice produced a detectable anti-HBs response in the soluble control pre-IgG group, whereas no mice receiving alum-precipitated pre-IgG prior to HBsAg produced a detectable anti-HBS response at a 1:5 serum dilution (Table 2). In this experiment, the anti-HBs response was measured by both a commercial RIA which detects IgM and IgG anti-HBs and a solid-phase RIA. For the remaining experiments the solid-phase RIA was used for titration of the mouse antisera, because this test is more sensitive, less expensive, and requires lower quantities of serum (50 µl versus 200 µl) when compared to the AUSAB. It was noteworthy that primarily IgM anti-HBs was detected by an IgM type-specific RIA in the group of mice injected with the soluble anti-ID preparation. This was similar to the observations described above where the number of direct IgM anti-HBs PFU was enhanced by prior injection of soluble anti-ID.

## TABLE 2

### Comparison of Alum-Precipitated and Soluble
### Anti-Idiotype for Induction of Anti-HBs[1]

| First injection | Second injection | No. of mice | Anti-HBs response (mean± SEM)[2] | Micro-SPIRA | |
| --- | --- | --- | --- | --- | --- |
| | | | | Anti-HBs IgG[3] | Anti-HBs IgM[4] |
| Anti-ID alum. ppt. | HBsAg | 4 | 487.5 ± 315.0 | 4938 | 1250 |
| Anti-ID soluble | HBsAg | 4 | 72.5 ± 50.0 | 86 | 1000 |
| Pre-rabbit IgG alum. ppt. | HBsAg | 4 | <5.0[5] | <5.0 | <5.0 |
| Pre-rabbit IgG soluble | HBsAg | 4 | 6.25 ± 1.08 | 30 | 50 |

[1]   Each group of mice received 40 µg of anti-ID or pre-IgG on day 0, followed by 6 µg of HBsAg on day 14, all by the intraperitoneal route.  Mice were bled on day 26.

[2]   The values are the reciprocal dilution of antisera which gave an endpoint S/N of 2.1 as measured by AUSAB.

[3]   The mean value of reciprocal dilution of antisera which gave an endpoint S/N of 2.1 as measured by solid-phase RIA using $^{125}$I-labeled goat anti-mouse γ-chain specific antiserum.

[4]   The mean value of reciprocal dilution of antisera which gave an endpoint S/N of 2.1 as measured by solid-phase RIA using $^{125}$I-labeled rabbit anti-mouse µ-chain specific antiserum.

[5]   All mice were negative for anti-HBs at a serum dilution of 1:5.

0110706

In the second set of experiments, the optimal time interval between a primary injection of alum-precipitated anti-ID antibodies and a subsequent HBsAg inoculum for enhancing the anti-HBs response was determined. Only IgG anti-HBs was measured since alum-precipitated anti-ID generated a potent secondary IgG response to HBsAG. In the previous experiment, the alum-precipitated anti-ID treated mice generated a 4-fold higher IgG anti-HBs titer when compared to IgM (Table 2). Also, previous observations in reference to the secondary humoral anti-HBs response indicated that IgG was the predominant antibody class present between 10 and 20 days after HBsAg boost. For these reasons, serum was routinely obtained 12 days after HBsAg injection and only IgG anti-HBs was assayed in the remainder of the study. Based on the data presented in Table 3, the optimal anti-HBs response occurred when HBsAg was given 14 days after priming with anti-ID. In each instance, groups of mice that received anti-ID antibodies prior to HBsAg generated a higher anti-HBs response when compared to the mice given pre-IgG. The extremely high standard errors of the mean table in Table 3 resulted from both the small sample size (n = 4) and the individual variations in anti-HBs titer among mice.

TABLE 3

Optimal Time Interval Between Injection of

Anti-Idiotype and HBsAg for Induction of Anti-HBs[1]

| First Injection | HBsAg injection (no. of days after primary inoculation) | Anti-HBs response[2] (mean ± SEM) |
|---|---|---|
| Anti-ID | 7 | 130 + 32.1 |
|  | 14 | 4222 ∓ 1825.7 |
|  | 21 | 200 ∓ 64.6 |
| Pre-IgG | 7 | 5 + 5.0 |
|  | 14 | <5.0[3] |
|  | 21 | 30 + 11.5 |

[1]     Each group of 4 mice received 40 µg of alum-precipitated anti-ID or pre-IgG on day 0, followed by 6 µg of HBsAg on the days specified, all by the intraperitoneal route. Mice were bled 12 days after injection with HBsAg.

[2]     The values are the reciprocal dilution of antiserum which gave an endpoint S/N of 2.1 as measured by the IgG anti-HBs solid-phase RIA.

[3]     All mice were negative for anti-HBs at a serum dilution of 1:5.

The optimal quantity of anti-ID required for enhancement of the anti-HBs response was determined in the next experiment (Table 4). Based on the two-tailed Student's t-test, the greatest significant difference ($p < 0.001$) of the anti-HBs titer was noted in that group of mice inoculated with 50 µg of alum-precipitated anti-ID versus pre-IgG prior to HBsAg stimulation. Significant differences were also obtained in the groups of mice injected with 5 and 200 µg of anti-ID when compared to control groups inoculated with similar doses of pre-IgG. Although the mean anti-HBs titer was higher in the group treated with 50 µg of anti-ID when compared to the groups given 5 or 200 µg of anti-ID the difference was not statistically significant. The reasons for this higher mean anti-HBs titer again may have reflected the small sample size (n = 4) and the individual variation of mice. No significant difference was noted in the anti-HBs response between the groups of mice receiving 500 ng of either anti-ID or pre-IgG prior to HBsAg (P > 0.2). Also, the mean anti-HBs titer was lower when compared to groups of mice receiving higher quantities of anti-ID. Due to the heterogeneity of the variances among the groups receiving different antibody concentrations prior to antigenic stimulation, the reciprocal arithmetic mean anti-HBs titers were expressed as values to $\log_{10}$ to facilitate the use of the parametric Student's t-test rather than a nonparametric test (i.e., Kruskal-Wallis) for the calculations of significance. It is noteworthy that some mice which received pre-IgG before HBsAg produced an IgG anti-HBs response (Table 4). The reasons for such a response are not known. However, it was consistent throughout the different experiments among the individual groups treated with pre-IgG. Based on the data presented in Table 4, 50 µg of anti-ID was selected as the optimal dose for generating anti-HBs in further experiments.

TABLE 4

Effect of Dose of Alum-Precipitated Anti-Idiotype

for Induction of IgG Anti-HBs Using Micro-SPIRA[1]

| First injection | Second injection | Reciprocal arithmetic mean titer | $Log_{10}$ of the reciprocal arithmetic mean titer | Standard deviation[2] |
|---|---|---|---|---|
| 500 ng anti-ID[3] | HBsAg | 583 | 2.76 | 0.77 |
| 500 ng pre-IgG | HBsAg | 86 | 1.93 | 0.26 $(p > 0.2)$[4] |
| 5 µg anti-ID | HBsAg | 4938 | 3.69 | 0.32 |
| 5 µg pre-IgG | HBsAg | 66 | 1.82 | 0.88 $(p < 0.02)$ |
| 50 µg anti-ID | HBsAG | 9378 | 3.97 | 0.10 |
| 50 µg pre-IgG | HBsAg | 6 | 0.78 | 0.18 $(p < 0.001)$ |
| 200 µg anti-ID | HBsAg | 4344 | 3.64 | 0.62 |
| 200 µg pre-IgG | HBsAg | 30 | 1.48 | 0.25 $(p < 0.01)$ |

[1]    Each group of 4 mice received various concentrations of alum-precipitated antibodies on day 0, followed by 6 µg of HBsAg on day 14.  Mice were bled on day 26.

[2]    The standard deviation of the $log_{10}$ of the reciprocal arithmetic mean titer.

[3]    Several of the mouse antisera were negative at the dilution tested.  To facilitate the computations the reciprocal of the titer was considered 0.5, assuming that greater than 2-fold concentration of the sample would give a positive result.

[4]    Determined by the two-tailed Student's t-test.

Previously (see above), we demonstrated that BALB/c mice receiving anti-ID antibodies alone generated indirect IgG anti-HBs PFU.  These data indicated that anti-ID antibodies could induce an anti-HBs response without HBsAg stimulation.  Based on the information established from the above experiments (Tables 2-4), we attempted to

0110706

induce anti-HBs in the serum of BALB/c mice by injecting anti-ID alone. A statistically significant IgG anti-HBs response was generated in mice receiving two injections of 50 µg of alum-precipitated anti-ID when compared to mice given similar injections of pre-IgG ($p < 0.001$) (Table 5).

TABLE 5
Induction of IgG Anti-HBs Antibodies that Express
the Idiotype by Injection of Anti-Idiotype[1]

| First Injection | Second Injection | Reciprocal arithmetic mean titer | $Log_{10}$ of the reciprocal arithmetic mean titer | Standard deviation[2] | % inhibition of binding of the ID-anti-ID reaction[3] |
|---|---|---|---|---|---|
| Anti-ID | anti-ID | 1000 | 3.0 | 0.24 | 38-54 |
| | | | | $(p < 0.001)$ [5] | |
| Pre-IgG[6] | pre-IgG | <5 | 0.3 | 0.00 | 0-9 |

[1] Each group of 4 mice received two injections of either 50 µg of alum-precipitated anti-ID or pre-IgG antibodies, 14 days apart.  Mice were bled 12 days after the second injection.

[2] The standard deviation of the $log_{10}$ of the reciprocal arithmetic mean titer.  Four mice were injected in each group.

[3] All sera were tested for the ability to inhibit the ID-anti-ID reaction at a 1:10 dilution.

[4] The range of mean values obtained from triplicate samples.

[5] Determined by the two-tailed Student's t-test.

[6] All four mouse antisera were negative at the dilution tested.  To facilitate the computations the reciprocal of the titer was considered to be 0.5, assuming that greater than 2-fold concentration of the sample would give a positive result.

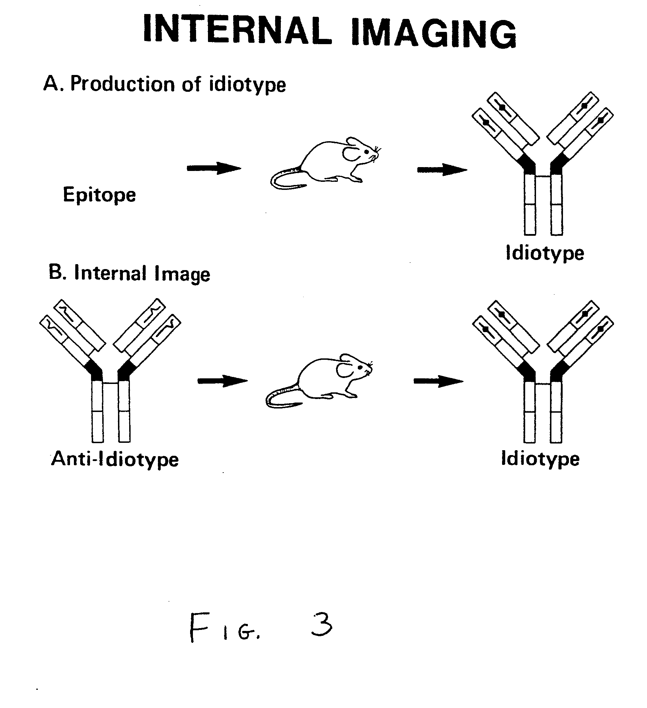

A mean anti-HBs titer of 1:1000 was obtained with 4 mice receiving anti-ID. Conversely, no anti-HBs was detected in 4 mice injected with control pre-IgG at a 1:5 serum dilution. These data indicate that anti-ID alone can produce detectable humoral anti-HBs in BALB/c mice. Figure 3. Analysis of the anti-ID induced anti-HBs demonstrated the expression of the interspecies ID (Table 5). In this regard, four sera containing anti-HBs generated by anti-ID induction inhibited the ID-anti-ID reaction from 38 to 54%, whereas less than 10% inhibition was obtained with the non-anti-HBs containing sera from the 4 mice injected with pre-IgG. In addition, all four preimmune sera from the mice that produced anti-HBs by anti-ID injection inhibited the binding of the ID to its anti-ID antiserum by less than 10%. Together, these data indicate that in vivo administration of anti-ID antibodies in BALB/c mice induced anti-HBs that expressed a similar interspecies ID. This interspecies ID was also detected in anti-HBs positive mice that received either anti-ID or pre-IgG prior to HBsAg (Table 6).

### TABLE 6

Expression of the Interspecies Idiotype in Mouse Anti-HBs[1]

| First injection | Second injection | Anti-HBs titer[2] | % inhibition of the idiotype-- anti-idiotype reaction |
|---|---|---|---|
| Anti-ID | HBsAg | 6250 | 52 |
| Anti-ID | HBsAg | 250 | 32 |
| Anti-ID | HBsAg | 6250 | 61 |
| Anti-ID | HBsAg | 6250 | 60 |
| Pre-IgG | HBsAg | 250 | 28 |
| Pre-IgG | HBsAg | >5 | 2 |
| Pre-IgG | HBsAg | >5 | 5 |
| Pre-IgG | HBsAg | 25 | 19 |

[1]   Mice received either 5 µg of alum-precipitated anti-ID or pre-IgG on day 0, followed by 6 µg of HBsAg on day 14.  Mice were bled on day 26.

[2]   Reciprocal dilution of antisera that gave a positive (S) to negative (N) cpm ratio of 2.1.

It was of interest that anti-HBs from the four mice receiving anti-ID prior to HBsAg inhibited the idiotype-anti-idiotype reaction to a greater degree (32-61%) when compared to anti-HBs from two of the four mice given pre-IgG prior to HBsAg.  Although this greater amount of inhibition of the idiotype-anti-idiotype reaction may have reflected higher concentrations of anti-HBs in the mice treated with anti-ID, previous studies indicated that the level of interspecies ID inhibition was not related to the anti-HBs titer. The two mice that received pre-IgG prior to HBsAg and failed to produce a detectable anti-HBs response inhibited the idiotype-anti-idiotype reaction by <5%.

Serologically, HBsAg contains a group-common antigenic reactivity referred to as the a determinant and two sets of allelic subtype determinants d or y and w or r. In this regard, four possible HBsAg subtypes have been recognized: adw, ayw, adr and ayr. The antibody specificity of the anti-ID induced anti-HBs response in terms of which HBsAg determinants were recognized was determined by radioimmunoassay (RIA) using microtiter plates coated with HBsAg subtypes adw, ayw, and adr. Each of the four anti-ID induced anti-HBs bound equally well the three different HBsAg subtypes (Table 7) indicating that the reactivities of the anti-ID induced anti-HBs were directed to the a determinant. (See also Figure 1). This substantiates our previous observation in that the binding of anti-ID antibodies was equally inhibited by preincubating the anti-HBs ID with three of the four HBsAg subtypes, indicating that the a determinant was responsible for the induction of the ID. The fact that the anti-ID induced anti-HBs recognized the group-specific a determinant of HBsAg lends evidence that anti-ID antibodies can induce protective immunity against infectious HBV. In this light, it was demonstrated that antibodies directed against the a determinant confer protection in humans against HBV infection. No significant binding to the three HBsAg subtypes was obtained with sera from the 4 mice that received two injections of pre-IgG alone.

TABLE 7

HBsAg Specificity of the Anti-ID Induced Anti-HBs

| First injection | Second injection | Reciprocal dilution of antisera tested | S/N ratio[1] | | |
|---|---|---|---|---|---|
| | | | adw | ayw | adr |
| Anti-ID | Anti-ID | 50 | 8.3 | 7.8 | 7.7 |
| | | 1250 | 1.8 | 1.5 | 1.6 |
| Anti-ID | Anti-ID | 50 | 8.0 | 7.8 | 7.8 |
| | | 1250 | 2.6 | 2.3 | 2.0 |
| Anti-ID | Anti-Id | 50 | 7.9 | 8.4 | 7.7 |
| | | 1250 | 3.6 | 3.3 | 4.4 |
| Anti-ID | Anti-ID | 50 | 7.9 | 7.9 | 8.2 |
| | | 1250 | 2.7 | 4.1 | 4.0 |
| Pre-IgG[2] | Pre-IgG | 10 | <1.8 | <1.6 | <1.8 |

[1] The calculation of the positive (S) to negative (N) ratio was done as previously described.

[2] These data represent a composite of 4 mice that each received 2 injections of pre-IgG.

A major problem associated with synthetic HBsAg peptides involves their relatively weak immunogenicity. A weak primary antibody response to HBsAg (anti-HBs) was induced in mice inoculated with an uncoupled cyclic synthetic peptide containing amino acid sequences 122-137 (peptide 1). This immune response was not boosted upon subsequent injections of the uncoupled peptide. Weak anti-HBs induction has characterized the responses in animals inoculated with other HBsAg peptides, even when coupled to various carrier proteins to increase immunogenicity. To our knowledge, the only study that has induced high-titered anti-HBs to synthetic peptides comparable to that of HBsAg particles involved the injection of mice with an alum-precipitated cyclic peptide 1 that was linked to tetanus toxoid.

The idea that the immune response to an antigen can be regulated via an idiotype-anti-idiotype network was first proposed by Jerne (Reference No. 3). In the study of the anti-HBs response in mice, we examined the effect of the in vivo administration of anti-idiotype antibodies prior to a single injection of cyclic peptide 1. Based on the data shown in Table 8, BALB/c mice treated with anti-idiotype antibodies prior to injection of cyclic peptide 1 generated a higher mean anti-HBs titer (38.6 as opposed to 4.0) when compared to mice injected with IgG from unimmunized rabbits. In confirmation of our previous observation at the cellular level, mice treated with anti-idiotype antibodies prior to HBsAg produced a higher mean anti-HBs titer when compared to groups of mice receiving pre-IgG before HBsAg. It is noteworthy that mice given anti-idiotype and peptide 1 had comparable anti-HBs titers with mice receiving pre-IgG and complete HBsAg (38.6 compared to 34.0; Table 1). These data indicate that anti-idiotype in conjunction with peptide 1 can induce anti-HBs titers comparable to a single injection of whole HBsAg particles. The results indicate that anti-idiotype antibodies can prime the immune system of mice for either a synthetic HBsAg peptide or intact HBsAg particles.

## TABLE 8

### Priming the anti-HBs response by prior injection of anti-idiotype antibodies

| First injection | Second injection | No. of mice | Anti-HBs response (mean+SEM)* |
|---|---|---|---|
| Pre-IgG | peptide 1 | 6 | 4.0 + 1.9 |
| Anti-idiotype | peptide 1 | 7 | 38.6 + 9.6 |
| Pre-IgG | HBsAg | 5 | 34.0 + 8.7 |
| Anti-idiotype | HBsAg | 6 | 10,416 + 306 |

All mice received 50 µg of either alum-precipitated anti-idiotype or control, preimmune rabbit IgG on day 0, followed by 50 µg of peptide 1 or 6 µg of HBsAg on day 14, all by the intraperitoneal route. Serum was obtained on day 30, and the reciprocal of the endpoint dilution which gave an arbitrary positive-to-negative cpm ratio of 2.1 was determined by radio-immunoassay as previously described.

*Mean of reciprocal endpoint titer (+ standard error of the mean) in the responding animals.

In Table 9 the kinetics of the anti-HBs response are reported. Only two of six mice produced a detectable anti-HBs response when treated with pre-IgG prior to the peptide, whereas all seven anti-idiotype treated mice generated detectable anti-HBs. The anti-HBs response peaked at 16 days following antigen exposure in each treatment group of mice. Comparison of the groups of mice given anti-idiotype and peptide 1 with pre-IgG and HBsAg indicated the anti-HBs titer was similar throughout the immune response even though the anti-idiotype treated group required 16 days for 100% responsiveness. The reason the mean anti-HBs titer was lower at day 6 in the mice given anti-idiotype and HBsAg

0110706

when compared to pre-IgG prior to HBsAg is not known (10 as opposed to 26). However, by day 16 mice injected with anti-idiotype antibodies prior to HBsAg had the highest anti-HBs response when compared to all other groups. These data are consistent with the fact that anti-idiotype antibodies are able to prime the immune system of mice prior to antigenic stimulation. These data further support that anti-idiotype antibodies can manipulate the immune response.

TABLE 9

Kinetics of the anti-HBs response

| Injection | | Days after final injection | | | |
|---|---|---|---|---|---|
| First | Second | | 6 | 16 | 26 |
| Pre-IgG | peptide 1 | 0/6* | 1/6 (2.5)** | 2/6 (4.0) | 1/6 (2.5) |
| Anti-idiotype | peptide 1 | 3/7 (5.7) | 6/7 (8.5) | 7/7 (38.6) | 7/7 (27.1) |
| Pre-IgG | HBsAg | 5/5 (10.0) | 5/5 (26.0) | 5/5 (34.0) | 5/5 (34.0) |
| Anti-idiotype | HBsAg | 4/6 (6.6) | 6/6 (10.0) | 6/6 (10,416) | 6/6 (1,750) |

All mice received 50 µg of either alum-precipitated anti-idiotype or control preimmune rabbit IgG on day 0, followed by 50 µg of peptide 1 or 6 µg of HBsAg on day 14, all by the intraperitoneal route.

\* The number of mice producing a detectable anti-HBs response over the total number of mice injected.

\*\* The numbers in parentheses are the reciprocal of the mean anti-HBs endpoint titer in the responding animals.

The theoretical implications for the use of anti-ID antibodies as vaccines for infectious agents has been previously proposed and discussed elsewhere (References 3-5). To our knowledge, the only experimental evidence where anti-ID can induce protective immunity against an infectious agent has been reported for African trypanosomiasis in mice (References 22-23). A second possibility of anti-ID induced immunity appears with HBV. Previous studies have demonstrated that anti-HBs is responsible for protection against challenge or reinfection with HBV. The fact that anti-HBs can be

0110706

induced by injection of anti-ID alone and this anti-HBs expresses an idiotypic determinant that is shared by anti-HBs produced in humans naturally infected with HBV suggests the potential for the use of anti-ID vaccines for HBV infections. In addition, the anti-HBs also recognized the group-specific a determinant of HBsAg, which is the epitope that induces protective immunity against HBV. This possibility must await further testing, since only chimpanzees and humans can be infected with human HBV. Alternatively, anti-ID could be used in conjunction with HBsAg to potentiate the anti-HBs response to a single injection of HBsAg by priming the host's immune system prior to antigenic stimulation. These studies using mice to induce anti-HBs by injection of anti-ID represent an attempt to understand the humoral immune response to HBsAg via modulation of an ID network. It is not known whether the anti-HBs produced in the modulation process described herein resulted from the expression of clones secreting an Ab-3 (anti-anti-ID antibodies) which expresses the ID and binds antigen or represents an internal image of HBsAg in which classic antigenic stimulation does not occur.

Thus, anti-idiotype antibodies are useful for priming the immune response to infectious agents. In the present invention, anti-idiotype antibodies prime the immune system of mice prior to stimulation with complete HBsAg. In addition, prior inoculation of the anti-idiotype has enabled the synthetic cyclic peptide 122-137 to elicit an anti-HBs response comparable to that obtained with a single injection of whole HBsAg particles. Recently, small peptides have been used to prime the immune system of experimental animals for the later induction of neutralizing antibodies to poliovirus (Reference 24). We have now shown that anti-idiotype antibodies may prime the host immune system to potentiate a protective immune response to a weak vaccine, and perhaps it may directly do the same to an infectious agent. This property may be sufficient to protect against infectious agents with long incubation periods and with propensities to set up persistent infections, the most notable of which is type B viral hepatitis.

The present invention, therefore, is well adapted to carry out the objects and attain the ends and advantages mentioned as well as others inherent therein. While a presently preferred embodiment of the invention has been given for the purpose of disclosure, numerous changes in compositions and steps of the methods will be readily apparent to those skilled in the art and which are encompassed within the spirit of the invention and the scope of the appended claims.

CLAIMS

1.     A composition of matter comprising a precipitated anti-idiotype antibody.

2.     The composition of claim 1 including an adjuvant vehicle.

3.     The composition of claim 1 wherein the precipitate anti-idiotype antibody is selected from the group consisting of antisera raised in a heterologous species or mouse monoclonal, rat monoclonal, or human monoclonal and antibody fragments thereof.

4.     The composition of claim 1 wherein the precipitated anti-idiotype antibody is specific for modulating immune response to HBsAg.

5.     An immunizing preparation for eliciting production of antibody to hepatitis B surface antigen comprising an anti-idiotype antibody specific to the a determinant of HBsAg.

6.     The immunizing preparation of claim 5 wherein the anti-idiotype antibody is specific to the a determinant of HBsAg and subtypes adw, ayw and adr thereof.

7.     An anti-idiotype antibody adsorbed on a solid particulate material.

8.     A method for modulating the immune response to an infectious viral agent or a viral agent that induces tumors or a viral-associated auto-immune syndrome in a host comprising injecting an anti-idiotype antibody into the host.

9.     The method of claim 8 wherein the viral agent is hepatitis B virus or the tumor induced is hepatitis B virus associated primary hepatocellular carcinoma.

10. The method of claim 8 wherein the viral agent is hepatitis B virus and the antibodies induced are anti-HBs.

11. The method of claim 10 wherein the anti-HBs is directed against the a determinant of HBsAg to inhibit interspecies anti-HBs idiotype-anti-idiotype reaction.

12. A method for modulating the immune response to hepatitis B surface antigen in a host comprising injecting anti-idiotype antibodies into the host and inducing anti-HBs.

13. The method of claim 12 wherein the anti-HBs is directed against the a determinant of HBsAg to inhibit interspecies anti-HBs idiotype-anti-idiotype reactions or other purative anti-HBs idiotype-anti-idiotype reactions.

14. The method of claim 12 wherein, after injection of the anti-idiotype antibodies into the host, the host is inoculated with native HBsAg.

15. The method of claim 12 wherein, after injection of the anti-idiotype antibodies into the host, the host is inoculated with an HBsAg synthetic peptide or HBsAg produced by recombinant DNA technology.

16. The method of claim 12 wherein the HBsAg syntehtic peptide is a cyclic synthetic peptide containing amino acid residues 117-137 and 122-137 in conjugated or unconjugated forms.

# IDIOTYPES

**A.** Reaction of idiotype with specific epitope (HBs peptide)

HBs
Peptide

Anti-HBs

Immune
Complex

**B.** Blocking of idiotype with anti-idiotype antibody

Anti-HBs

Anti-CId

Free
Peptide

Figure 1

0110706

# IDIOTYPES

A. Reaction of idiotype with specific epitope (HBs peptide)

B. Blocking of idiotype with HBs antigen

FIG. 2

# INTERNAL IMAGING

**A. Production of idiotype**

**Epitope**

**Idiotype**

**B. Internal Image**

**Anti-Idiotype**

**Idiotype**

FIG. 3

Figure 4

Kinetics of the anti-HBs plaque-forming units (PFU) response
in mice injected with a 40 µg-dose of antibody to idiotype
followed 7 days later with an injection of 5 µg of HBsAg.
The bars represent the mean PFU (open bar, direct PFU;
hatched bar, indirect PFU) per spleen of three mice and the
brackets represent the range observed. No direct or indirect
plaques were detected with uncoated or ovalbumin-treated SRBC
at any time interval.